(19) Europäisches Patentamt
European Patent Office
Office européen des brevets

(11) Publication number: 0 318 898
A2

(12) # EUROPEAN PATENT APPLICATION

(21) Application number: 88119813.9

(22) Date of filing: 28.11.88

(51) Int. Cl.4: C12P 17/12 , //(C12P17/12, C12R1:465)

(30) Priority: 28.11.87 JP 300599/87

(43) Date of publication of application: 07.06.89 Bulletin 89/23

(84) Designated Contracting States: BE CH DE ES FR GB IT LI NL

(71) Applicant: NIPPON SHINYAKU COMPANY, LIMITED
14, Kisshoin Nishinosho Monguchicho
Minami-ku Kyoto-shi Kyoto 601(JP)

(72) Inventor: Sugiyama, Makoto
6-23 Otowa Otsudecho
Yamashina-ku Kyoto(JP)
Inventor: Ezure, Yoji
2-21-22 Nogohara
Otsu-shi 520-21(JP)
Inventor: Ojima, Nobutoshi
50-9 Miyakecho
Moriyama City Shiga Pref.(JP)
Inventor: Konno, Kiyotaka
2-9-9-304 Oharano Nishisakaidanicho
Nishikyo-ku Kyoto(JP)
Inventor: Seto, Takashi
Room 202 Entopia Saga 9-4 Sagashakado
Monzen Setogawacho Ukyo-ku Kyoto(JP)
Inventor: Nakamura, Teruya
831-6 Nomuracho
Kusatsu City(JP)
Inventor: Itoh, Manabu
2-23 Tamanoura
Otsu City(JP)

(74) Representative: Wey, Hans-Heinrich, Dipl.-Ing.
Patentanwälte Wey & Partner
Widenmayerstrasse 49
D-8000 München 22(DE)

(54) **Method for manufacturing 1-deoxymannojirimycin.**

(57) 1-Deoxymannojirimycin represented by the general formula [I] which is a potent inhibitor against α-mannosidase and affects the biosynthesis of glycoprotein and inhibits sugar chain processing and is thus expected to be an immune regulator, can be manufactured by a method which comprises culturing a microorganism belonging to the genus Streptomyces and capable of producing 1-deoxymannojirimycin in a medium, and isolating 1-deoxymannojirimycin from the culture broth.

EP 0 318 898 A2

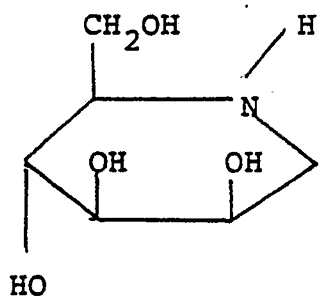

[I]

## Method for Manufacturing 1-Deoxymannojirimycin

The present invention relates to a method for manufacturing 1-deoxymannojirimycin which is a potent inhibitor against α-mannosidase.

More particularly, the present invention relates to a method for manufacturing 1-deoxymannojirimycin which comprises culturing a microorganism belonging to the genus Streptomyces and capable of producing 1-deoxymannojirimycin in a medium, and isolating from the culture 1-deoxymannojirimycin represented by general formula [I] from the culture broth.

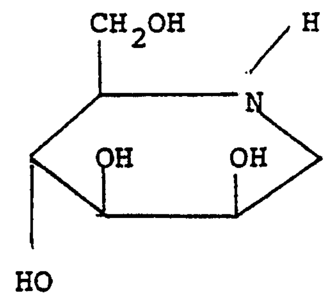

[I]

1-Deoxymannojirimycin affects biosynthesis of glycoprotein and inhibits sugar chain processing and is thus expected to be an immune regulator. Further, 1-deoxymannojirimycin is a starting material for synthesis of N-substituted derivative of 1-deoxymannojirimycin or swainsonine and derivatives thereof and hence, its utility has been expected.

1-Deoxymannojirimycin is a known compound and prepared by a known methods which comprise isolating from beans (J. Chem. Soc., Chem. Commun., 1979, 977) or by a known synthetic method (Carbohydrate Research, 164, 141-148 (1987)), etc. (Problems to be solved by the Invention)

In obtaining the compound of the present invention by the above methods, the yield was poor and many steps were required so that many disadvantages were involved from an economical viewpoint. The present inventors made extensive investigations to solve these problems.

As a result of investigations on a method for manufacturing 1-deoxymannojirimycin by fermentation over a wide range, the present inventors have found the fact that a microorganism belonging to the genus Streptomyces can produce 1-deoxymannojirimycin and have come to accomplish the present invention.

A representative example of the microorganism in accordance with the present invention is Streptomyces lavendulae SEN-158 isolated by the present inventors from a soil of Sapporo City, Japan (hereafter referred to as "SEN-158 strain").

SEN-158 strain has been deposited in the Fermentation Research Institute of the Agency of Industrial Science & Technology of Japan under Accession Number of FERM P-4301. Its bacteriologial properties are described in Japanese Published Unexamined Patent Application No. 084094/1979. Further this strain has been deposited in the American Type Culture Collection, Rockville Md., under Accession Number of ATCC 31434.

In the present invention, any strain other than the SEN-158 strain is usable as far as it belongs to the genus Streptomyces and is capable of producing 1-deoxymannojirimycin. Further, artificial mutants obtained by applying to these strains variation means conventionally used such as a treatment for irradiating with ultraviolet rays or $^{60}Co$, etc., a variation treatment with a variation inducer such as nitrogen mustard, azaserine, nitric acid, nitrosoguanidine or 2-aminopurine, etc., transduction, transformation, cell fusion, etc. and naturally occurring variants are also suited for the purpose of the present invention.

The present invention can be practiced by using the 1-deoxymannojirimycin-producing strain and culturing the strain in a conventional manner for culturing Actinomycetes.

A medium can be liquid or solid but shake culture or submerged culture in liquid medium can ordinarily be used.

The medium can be any one that is suited for growth of Actinomycetes and capable of producing 1-deoxymannojirimycin.

As the carbon sources, there can be used glucose, galactose, mannitol, sucrose, maltose, glycerine, dextrin, starch, or the like.

As the nitrogen sources, there can be used soybean powders, peptone, yeast extract, meat extract, corn steep liquor, ammonium chloride, ammonium sulfate, ammonium nitrate, urea, etc.

In addition, supplementation of sodium chloride, potassium chloride, calcium carbonate, various phosphates or the like in a suitable amount can provide good results. Furthermore, a suitable amount of iron, magnesium, etc. may also be added.

If necessary, organic or inorganic compounds, vitamins, etc. can be supplemented to accelerate growth of the microorganisms used or production of 1-deoxymannojirimycin.

In case that foaming is remarkable during fermentation, a defoaming agent may be appropriately added.

Conditions for incubation such as a pH value of the medium, incubation temperature and the like can be appropriately varied within such a range that produces 1-deoxymannojirimycin. For example, in shake culture or submerged culture, it is desired to culture at pH of from 6 to 9 at an incubation temperature of from approximately 20 to 35° C, preferably 25 to 30° C.

A time period for incubation varies depending upon scale of culture and other conditions but it is generally sufficient for 2 to 20 days.

After incubation, the cells are separated and the product is isolated from the obtained culture broth and purified.

In order to isolate and purify the substance of the present invention from the culture brota, conventional technique used for isolating and purifying microorganism metabolites from the culture broth can be used.

For example, adsorption and desorption with various adsorbents (e.g., silica gel, alumina, activated charcoal, ion exchange resin, etc.), chromatography, partition chromatography, etc., can be used singly or in combination.

Upon practice of the present invention, moranoline represented by structural formula [II] is often co-present in the culture broth of Actinomycetes with the substance of the present invention.

In such a case, the substance of the present invention may be isolated by the aforesaid fractionation; alternatively, the substance of the present invention may also be obtained by applying such a chemical treatment that does not react with moranoline but reacts only with the substance of the present invention, to the state that the substance of the present invention is mixed with moranoline, leading the substance of the present invention to its derivative, isolating only the substance of the present invention in a form of its derivative by means of extraction with a solvent, etc. and then applying an appropriate chemical treatment to the substance of the present invention.

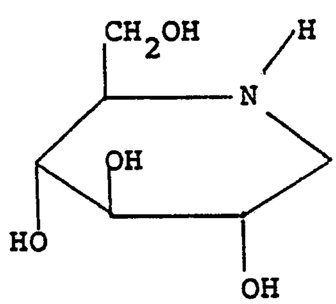

[II]

For example, the substance of the present invention is led to its N-tert-butoxycarbonyl derivative or N-benzyl derivative in a state mixed with moranoline and then the derivative is converted into the 2,3-isopropylidene derivative. In this case, moranoline does not form the isopropylidene derivative but only the substance of the present invention produces the isopropylidene derivative. Therefore, by performing partition with chloroform and water in the state that both are mixed, only the N-tert-butoxycarbonyl-2,3-isopropylidene derivative of the substance of the present invention can be partitioned in chloroform. Then, this protective group is removed thereby to obtain the substance of the present invention alone as the objective product.

Hereafter the present invention is described in more detail by referring to examples below.

## Example 1

In a 500 ml Erlenmeyer flask, 100 ml of medium (8% of soluble starch, 1% of soybean powders, 1% of yeast extract, 0.05% of potassium chloride, 0.05% of magnesium sulfate, 0.5% of sodium chloride and

0.2% of sodium nitrate, pH 7.2) was charged and sterilized. Several platinum loops of SEN-158 strain were inoculated to the medium followed by shake culture at 27° C for 3 days to give a seed culture solution. This seed culture solution, 300 ml, was inoculated on 15 liters of fermentation medium (medium having the same composition as in the seed medium described above) charged in a 30 liter volume jar fermenter followed by submerged culture at 27° C for 11 days.

As a defoaming agent, NISSAN DISFOAM CB-442 was used; an aeration rate and an agitation speed were 20 liters/min. and 300 rpm, respectively.

After 12.9 liters of the obtained culture broth was centrifuged at 9000 rpm for 20 minutes, the obtained supernatant was passed through a column packed with strongly acidic ion exchange resin Dowex 50W x 2 ($H^+$) (1 liter). After thoroughly washing with water, elution was performed with 1 N ammonia water.

The eluate was concentrated under reduced pressure. The concentrate was passed through strongly basic ion exchange resin Diaion SA-11A ($OH^-$) (500 ml) followed by washing with water. The eluate was combined with the washing liquid. The mixture was concentrated under reduced pressure. The concentrate was again passed through a column packed with Dowex 50W x 2 ($H^+$) (300 ml). After washing with water, the eluate was subjected to chromatography using 0.5 N ammonia water.

The fraction containing the product was concentrated to dryness under reduced pressure and the concentrate was dissolved in methanol. The solution was subjected to chromatography through a Sephadex LH-20 column (200 ml) and developed with methanol. The fraction containing the product was concentrated to dryness under reduced pressure and the residue was dissolved in methanol with heating. The solution was allowed to stand and the formed crystals were collected. The crystals were recrystallized from methanol to give 140 mg of 1-deoxymannojirimycin.
Melting point: 187-188° C

| Elemental analysis ($C_6H_{13}NO_4$) | | | | | | |
|---|---|---|---|---|---|---|
| Calcd. (%) | C: | 44.16 | H: | 8.03 | N: | 8.58 |
| Found (%) | C: | 44.17 | H: | 8.08 | N: | 8.63 |

Specific rotary power $[\alpha]_D^{24}$ = -45.49° (C = 1.02, water)
Hydrochloride of 1-deoxymannojirimycin:
$^{13}C$-NMR ppm; ($D_2O$, internal standard; methanol 49.8 ppm)
48.4 58.9 61.2 66.5 66.7 73.2
$^1H$-NMR ppm; ($D_2O$, internal standard; DSS)
2.87 (1H, m), 3.04 (1H, dd, J = 1.0, 15Hz), 3.25 (1H, dd, J = 3.0, 15Hz), 3.60-3.95 (4H, m), 4.14 (1H, m)

Example 2

The culture solution (1 liter) containing moranoline and 1-deoxymannojirimycin was passed through a column packed with strongly acidic ion exchange resin Dowex 50W x 2 ($H^+$) (100 ml). After thoroughly washing with water, elution was performed with 0.5 N ammonia water. The solvent was distilled off under reduced pressure and the residue was dissolved in water. The solution was passed through strongly basic ion exchange resin Diaion SA-11A ($OH^-$) (100 ml) followed by thoroughly eluting with water. The solvent was distilled off under reduced pressure and the residue was dissolved in 2 ml of water. Triethylamine, 3,2 ml, and a solution of 23.2 g of 2-tert-butoxycarbonylthio-4,6-dimethylpyrimidine previously dissolved in 2 ml of dioxane were added to the solution. The mixture was reacted at 60° C overnight.

The reaction solution was freeze dried and dioxane was distilled off. The obtained syrup was subjected to silica gel column chromatography (Wakogel C-200, eluting solution: a, 100:1, b, 50:1, c, 20:1; methylene chloride : methanol). A mixture of N-Boc derivative of moranoline and N-Boc derivative of 1-deoxymannojirimycin was obtained from Eluate c.

After the solvent was distilled off under reduced pressure, the residue was dissolved in 10 ml of methanol and 200 ml of dry acetone was added to the solution. Further 2 g of anhydrous ferric chloride was added to the mixture followed by reacting at room temperature for 2 days.

10% Potassium carbonate aqueous solution was added to the reaction mixture. After the pH was adjusted to 7 to 8, 100 ml of water was added to the reaction solution followed by partition with chloroform.

The chloroform layer was collected and the solvent was distilled off under reduced pressure. Then, the residue was dissolved in 40 ml of methanol and 20 ml of 1 N hydrochloric acid was added to the solution. The mixture was refluxed for 5 hours. After methanol was distilled off, the residue was passed through

Diaion SA-11A ($OH^-$) (100 ml) followed by thoroughly washing with water. The eluate was combined with the washing liquid. The mixture was passed through a column packed with Dowex 50W x 2 ($H^+$) (100 ml). After thoroughly washing with water, elution was performed with 0.5 N ammonia water. After the eluate was subjected to chromatography using 0.5 N ammonia water. After the solvent was distilled off under reduced pressure, the residue was crystallized from methanol to give 0.5 g of 1-deoxymannojirimycin.

## Claims

1. A method for manufacturing 1-deoxymannojirimycin which comprises culturing a microorganism belonging to the genus Streptomyces and capable of producing 1-deoxymannojirimycin in a medium, and isolating 1-deoxymannojirimycin from the culture broth.